# EUROPEAN PATENT SPECIFICATION

(11) **EP 1 177 164 B1**
(45) Date of publication and mention of the grant of the patent: **29.09.2004**
(21) Application number: 00931071.5
(22) Date of filing: 25.04.2000
(51) Int. Cl.: C07C 69/95, C07C 67/52, C07C 69/00

(54) **A PROCESS FOR THE PURIFICATION OF DIACEREIN**
VERFAHREN ZUM REINIGEN VON DIACEREIN
PURIFICATION DE DIACEREINE

(30) Priority: 07.05.1999 IT MI990994
(43) Date of publication of application: 06.02.2002
(73) Proprietor: Synteco S.p.A., 27028 S. Martino Siccomario (IT)
(72) Inventor: SINISTRI, Monica, I-27028 San Martino Siccomario (IT); SINISTRI, Roberta, I-27028 San Martino Siccomario (IT)
(74) Representative: Minoja, Fabrizio, Dr.
(86) International application number: PCT/EP2000/003691
(87) International publication number: WO 2000/068179

(56) References cited:
- EP-A- 0 636 602
- WO-A-96/24572
- WO-A-98/56750

## Description

The present invention relates to an improved process for the purification of diacerein.

The most known process for the preparation of diacerein comprises acetylation of aloin to form acetylaloin (acetylbarbaloin) which is subsequently oxidized with chromic acid.

The subsequent purification process aims at obtaining a product substantially free from impurities and in particular at removing aloe-emodin, which is known to have mutagenic properties above 70 p.p.m. threshold values, as well as at obtaining a product having a chromium content below 50 p.p.m.

A number of methods for the purification of crude diacerein are known. EP-A- 96900687 discloses a process in which diacerein is suspended in a mixture of organic solvents and water, a solution is obtained by addition of a tertiary amine, impurities are filtered off, diacerein is reprecipitated as an alkali/alkaline-earth metal salt, which is then dissolved in water to obtain diacerein in slightly acidic medium. It has, however, been observed that solubilization of diacerein salt in water, prior to acidification with weak acids, induces partial deacetylation with consequent formation of one/two impurities causing a loss of the product titre of about 0.5 to 5%. The loss of titre (i.e. of purity) is statistically 2%.

On the other hand, it is known from chemical literature that the protection of hydroxy groups by acetylation is reversible, as hydrolysis occurs even in only slightly acidic or basic medium. In the case of diacerein, this remains stable when passing from neutral to acidic medium, but it undergoes fast deacetylation in even slightly basic medium: in particular, the basicity produced by diacerein sodium salt is sufficient to induce hydrolysis. Any subsequent reacetylation attempts with acetic acid, acetic anhydride or acetyl chloride are complex, incomplete and decrease yields.

EP 636,602 discloses a multi-step purification process, which is very complex and makes use of toxic solvents. More precisely, crude diacerein is dried and purified first by dissolution in methylerie chloride and triethylamine, then by precipitation by addition of acids. The product is separated by centrifugation, dried and crystallized from methylcellosolve, filtered, dried again and recrystallized from N,N-dimethylacetamide, filtered again and repeatedly washed with water to remove the solvent, which is otherwise difficult to remove by drying.

WO 98/56750 discloses a process for the preparation of diacerein which comprises acetylation of aloin with acetic anhydride, followed by oxidation of acetyl-barbaloin with chronic anhydride in acetic acid solution and by precipitation of crude diacerein from a water/ acetic acid solvent mixture.

WO96/24572 discloses a method for purifying diacetyl rhein which comprises the steps of (a) forming a suspension of diacetyl rhein in a mixture of an organic solvent and water, (b) adding a tertiary amine; (c) adding an alkali metal or alkaline-earth metal acid salt to the resulting solution; (d) performing hydrolysis in a weakly acid medium; and (e) recovering the purified diacetyl rhein by filtration.

It has now been found that highly pure diacerein can be obtained by simple crystallization of crude diacerein from an acetic anhydride/acetic acid mixture, or from only acetic anhydride, in the presence of small amounts of ethylenediaminotetraacetic acid (EDTA) to remove any chromium traces from the previous chromic oxidation of acetylbarbaloin, said crystallization being followed by dissolution in an acetone/triethylamine mixture and reprecipitation with diluted phosphoric acid, to thoroughly remove acetic acid.

According to the invention, crude diacerein is dissolved in 60-100 parts (v/w) of a hot acetic anhydride/acetic acid mixture in a ratio (v/v) ranging from 10:90 to 30:70; or in 10-25 volumes of only acetic anhydride. The hot solution is added with 0.05 to 0.2% by weight of EDTA, compared with diacerein, and the EDTA-chromium complex is filtered off while hot.

After that, the precipitate is cooled, filtered or centrifuged, washed with acetic acid, then with water, dissolved in 10-20 parts by volume of acetone/triethylamine in a 25:1 to 35:1 ratio and reprecipitated with a 4-8% phosphoric acid aqueous solution. After washing with water and drying, diacerein is obtained in 80-90% yields, having 99.7% minimum purity, containing less than 70 p.p.m. of aloe-emodin and no more than 15 p.p.m. of chromium, as determined by atomic absorption.

The process according to the invention is obviously also suitable for the purification of diacerein obtained by other routes, which do not involve chromic oxidation. In this case, the addition of EDTA is of course superfluous.

### Example 1

1 Kg of crude diacerein (obtained by chromic oxidation of acetylbarbaloin, see PCT/EP/03221) is dissolved in 80 litres of a hot acetic acid/acetic anhydride 88.75/11.25 mixture (v/v), 1 g of ethylenediaminotetraacetic acid is added thereto, insolubles (EDTA-chromium complex) are filtered off while hot, the solution is cooled to room temperature, filtered by suction and washed first with acetic acid, then with water. The precipitate is dissolved in 14 litres of acetone/triethylamine 31/1 (v/v) and reprecipitated by acidification with 130 litres of 6% aqueous phosphoric acid. After thoroughly washing with water and drying, 850 g of diacerein with 99.7% purity are obtained, containing < 50 p.p.m. of aloe-emodin and 15 p.p.m. of chromium.

### Example 2

The procedure of example 1 is followed, replacing the crystallization solvent with 17 litres of acetic anhydride. Furthermore, after eliminating the EDTA-chromium complex, the mixture is concentrated by distillation under reduced pressure to about 1/3 the starting volume, then it is worked up as described in example 1. A product with the same purity characteristics as the one of example 1 is obtained in an 83.6% yield.

## Claims

1. A process for the purification of crude diacerein, **characterized in that** diacerein is crystallized from a solvent consisting of acetic anhydride and acetic acid or from only acetic anhydride, the crystallized diacerein is then dissolved in an acetone/triethylamine mixture and reprecipitated with a phosphoric acid aqueous solution.

2. A process as claimed in claim 1, **characterized in that** diacerein is dissolved in 60-100 parts by volume of acetic anhydride/acetic acid mixture.

3. A process as claimed in claim 2, **characterized in that** the acetic anhydride/acetic acid ratio in the mixture ranges from 10:90 to 30:70.

4. A process as claimed in claim 1, **characterized in that** diacerein is dissolved in 10-25 parts by volume of the only acetic anhydride.

5. A process as claimed in the above claims, **characterized in that**, when crude diacerein has been obtained by chromic oxidation of acetylbarbaloin, the hot solution is added with 0.05 to 0.2% by weight, on diacerein weight, of ethylenediaminotetraacetic acid (EDTA) and that the EDTA-chromium complex is removed by filtration from the hot solution.

## Patentansprüche

1. Verfahren zur Reinigung von rohem Diacerein, dadurch charakterisiert, dass Diacerein aus einem aus Essigsäureanhydrid und Essigsäure bestehenden Lösungsmittel oder nur aus Essigsäureanhydrid kristallisiert wird, das kristallisierte Diacerein dann in einem Aceton/Triethylamin-Gemisch gelöst und mit einer wässrigen Phosphorsäure-Lösung ausgefällt wird.

2. Verfahren wie in Anspruch 1 beansprucht, dadurch charakterisiert, dass Diacerein in 60 bis 100 Volumenteilen Essigsäureanhydrid/Essigsäure-Mischung gelöst wird.

3. Verfahren wie in Anspruch 2 beansprucht, dadurch charakterisiert, dass das Essigsäureanhydrid/Essigsäure-Verhältnis im Gemisch im Bereich von 10 : 90 bis 30 : 70 liegt.

4. Verfahren wie in Anspruch 1 beansprucht, dadurch charakterisiert, dass Diacerein in 10 bis 25 Volumenteilen Essigsäureanhydrid gelöst wird.

5. Verfahren wie in den obigen Ansprüchen beansprucht, dadurch charakterisiert, dass, wenn rohes Diacerein durch Chromsäureoxidation von Acetylbarbaloin erhalten wurde, die heiße Lösung mit 0,05 bis 0,2 Gewichtsteilen, bezogen auf das Diacerein-Gewicht, Ethylendiamintetraessigsäure (EDTA) versetzt wird und dass der EDTA-Chromkomplex durch Filtration aus der heißen Lösung entfernt wird.

## Revendications

1. Procédé pour la purification de diacéréine brute, **caractérisé en ce que** la diacéréine est cristallisée à partir d'un solvant consistant en un mélange d'anhydride acétique et d'acide acétique ou à partir d'anhydride acétique seulement, la diacéréine cristallisée est ensuite dissoute dans un mélange acétoneltriéthylamine et reprécipitée avec une solution aqueuse d'acide phosphorique.

2. Procédé selon la revendication 1, **caractérisé en ce que** la diacéréine est dissoute dans 60 à 100 parties en volume de mélange anhydride acétique/acide acétique.

3. Procédé selon la revendication 2, **caractérisé en ce que** le rapport anhydride acétique/acide acétique dans le mélange va de 10:90 à 30:70.

4. Procédé selon la revendication 1, **caractérisé en ce que** la diacéréine est dissoute dans 10 à 25 parties en volume d'anhydride acétique seulement.

5. Procédé selon les revendications précédentes, **caractérisé en ce que**, lorsque la diacéréine brute a été obtenue par oxydation chromique d'acétylbarbaloïne, la solution chaude est additionnée de 0,05 à 0,2 % en poids (par rapport au poids de la diacéréine) d'acide éthylènediamineotétraacétique (EDTA), et **en ce que** le complexe EDTA-chrome est éliminé par filtration de la solution chaude.
